# EUROPEAN PATENT SPECIFICATION

(11) **EP 0 844 872 B1**
(45) Date of publication and mention of the grant of the patent: **20.12.2000**
(21) Application number: 96924043.1
(22) Date of filing: 10.07.1996
(51) Int. Cl.: A61K 9/72

(54) **USE OF AROMAS AND FLAVOURS TO INDUCE OR CHANGE EATING PATTERNS**
VERWENDUNG VON AROMEN UND GESCHMACKSSTOFFEN ZUR VERANLASSUNG ODER ÄNDERUNG DER ESSGEWOHNHEITEN
UTILISATION D'AROMES ET DE SAVEURS POUR CREER OU MODIFIER DES HABITUDES ALIMENTAIRES

(30) Priority: 14.07.1995 GB 9514469; 28.07.1995 GB 9515666; 06.03.1996 US 610555
(43) Date of publication of application: 03.06.1998
(73) Proprietor: The Aromacology Patch Company Limited, Petersfield, Hampshire GU31 4AD (GB)
(72) Inventor: PAUL, Elizabeth, Ilkley LS29 8PP (GB)
(74) Representative: Wharton, Peter Robert
(86) International application number: GB9601647
(87) International publication number: WO9703658

(56) References cited:
- WO-A-93/00115
- FR-A- 2 727 627

## Description

The present invention relates to a method and apparatus for inhaling aromas, perfumes, essences, odours and the like to inhibit, induce or change eating patterns, in particular cravings or desires for particular types of food or drink. The invention also encompasses the use of aromas and the like more generally for inhibiting, inducing or changing other aspects of human behaviour and conditions.

The invention is more particularly directed towards the regulation of bodyweight, in which the aroma is used to take away or at least significantly reduce the craving for sweet foods and drink, particularly chocolate.

It has been proposed that the inhalation of aromas can be of benefit in the control of bodyweight, but hitherto there has not been proposed any suitable, convenient way of delivering the aroma on a regular or continuous basis. Inhaler devices, such as those used by persons suffering from nasal congestion, have been proposed, but these have the disadvantage that they have to be carried around by the person using them, and can easily be forgotten or lost.

PCT Patent Specification No WO 93/00115 describes a multilayered laminate comprising a impermeable membrane layer, a reservoir layer incorporating an active compound, and a diffusion rate limiting membrane layer to restrict and hence control the rate of diffusion of the active compound therethrough.

It is therefore the aim of the present invention to provide a simple device for allowing the aroma to be easily inhaled as and when required or advised.

According to a first aspect of the present invention there is provided a device for use in delivering an aroma, the device comprising lint or similar material impregnated with the substance giving the desired aroma, the lint being incorporated into an adhesive plaster or patch, for application to the skin, wherein the surface of the plaster which is exposed when the plaster is worn includes a central hole therein, so that the aroma from the lint may escape therethrough.

Preferably, the surface of the lint which would otherwise come into contact with the skin is covered with an impervious layer, to prevent the aroma substance from entering the body through the skin.

Preferably, the plaster is made from permeable fabric, which allows the plaster to "breath", and the aroma to be given off through the fabric as well as from the edges of the plaster and/or the central hole.

Preferably, each plaster has releasably adhered thereto a pair of peelable "shields" which are removed to expose the adhesive.

Conveniently, each plaster is individually sealed in an impervious wrapper, to retain the aroma until use.

In the proposed use for bodyweight control, it is proposed that a fresh plaster is worn each day, on any convenient part of the body, such as the wrist, inner arm etc. The plaster would then be sniffed, either at regular intervals throughout the day, or whenever required.

As for the particular aroma to be used, the Applicant has found the aroma of vanilla particularly effective in the area of reducing cravings for sweet food and drink, particularly chocolate.

It has been found that when used as recommended, the desire for sweet foods, particularly chocolate or chocolate flavoured products, is greatly reduced and possibly even eliminated. It is anticipated that the plaster would eventually help to wean the user off chocolate and other sweet foods permanently.

The plaster, either alone or in conjunction with a suitable diet, can achieve acceptable weight loss over a period of time, amounting to an expected 2% of bodyweight per month even on a normal food intake.

The preferred substance used for this particular application is a 50/50 mixture of Floral Vanillin (Ref. C 6031/E Flora by A. Algto Ltd) and denatured ethanol, applied at the rate of 20 kilos per 250m roll of lint (one metre width).

However, it is envisaged that other aromas could be equally effective in both bodyweight control and for other purposed, such as inhibiting the desire to smoke, drink alcohol or other undesired and/or unhealthy habits. Certain aromas could be used to stimulate the appetite, increase sexual desire, and in some cases even relieve unpleasant physical or mental symptoms, for example suppressing nauseousness or helping to relieve depression or anxiety. In particular, lavender could be used to combat anxiety, and the aroma of pumpkin could be used to increase sexual desire. These aromas could either be natural extracts or the synthetic equivalents.

Whilst the aforementioned description refers to "an aroma", this is to be interpreted as meaning either a single, pure aroma, or a mixture of different aromas, diluted if necessary in either ethanol or any other suitable dilutant.

According to a second aspect of the present invention there is provided a method of manufacturing an adhesive plaster incorporating an aroma, for application to the skin, the method including the steps of:-
(a) impregnating lint or other similar material with a substance giving the desired aroma and allowing the impregnated lint to dry,
(b) applying the lint to an adhesive fabric backing,
(c) forming a series of holes in the adhesive fabric backing, and
(c) cutting the lint and fabric combination into individual plasters with a central hole in the fabric backing of each plaster.

For convenience, the lint is preferably impregnated whilst in the roll, and is then slit into the desired width, eg 1.5cm rolls which can then be used on conventional plaster making machinery.

The method also preferably includes the step of applying an impervious layer to the exposed surface of the impregnated lint. The free side of this impervious layer is then coated with a suitable adhesive, and the peelable "shields" applied.

Preferably, the method also includes the final step of sealing the plasters individually into an impervious wrapper.

Embodiments of the present invention will now be described, by way of example only, with reference to the accompanying drawings in which:-
Figure 1 is a plan view of a plaster;
Figure 2 is a side view of the plaster shown in Figure 1,;
Figure 3 is a plan view of an embodiment of the first aspect of the invention; and
Figure 4 is a side view of the plaster shown in Figure 3.

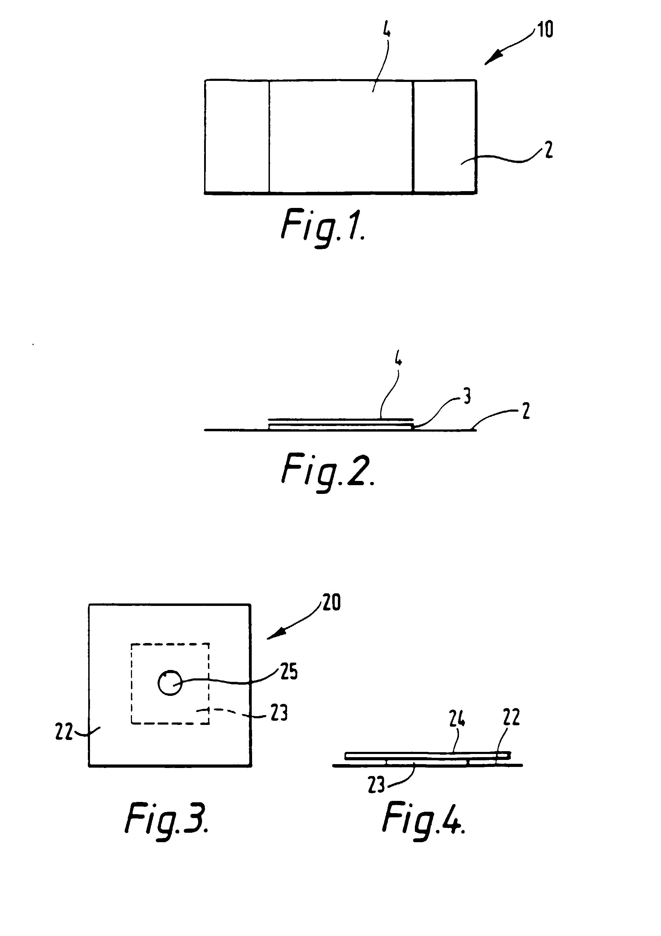

Referring to Figure 1 and 2 of the drawings, a plaster 10 comprises a self adhesive backing layer 2 (fabric or plastic) bearing in the centre thereof a piece of lint 3, the latter having being impregnated with the desired aroma substance, eg a mixture of vanillin and ethanol, the exposed surface of the lint being covered with an impervious layer 4 which prevents the substance from coming into contact with the skin when the plaster is worn.

When worn on the skin in a convenient place, using a fresh patch every day, the aroma given off by the plaster may be inhaled as required or as directed, to assist in, for example, bodyweight control either alone or in conjunction with dieting.

The individual plasters 10 are packed in individual impervious wrappers, in strips of seven. Four strips of seven plasters would be provided in a pack as 28 days' supply.

Whilst the plaster illustrated in Figures 1 and 2 is generally rectangular, in the embodiment according to the invention shown in Figures 3 and 4 the plaster 20 is generally square, with a smaller square of lint 23 located centrally, sandwiched between the adhesive backing layer 22 and the impervious layer 24 with a central hole 25 in the backing layer, to allow the aroma from the lint 23 to escape.

## Claims

1. A device for use in delivering an aroma, the device comprising lint or similar material impregnated with the substance giving the desired aroma, the lint being incorporated into an adhesive plaster or patch, for application to the skin, wherein the surface of the plaster which is exposed when the plaster is worn includes a central hole therein, so that the aroma from the lint may escape therethrough.

2. A device according to Claim 1 wherein the surface of the lint which would otherwise come into contact with the skin is covered with an impervious layer, to prevent the aroma substance from entering the body through the skin.

3. A device according to any of the preceding Claims wherein the plaster is made from permeable fabric, which allows the plaster to "breath", and the aroma to be given off through the fabric.

4. A device according to any of the preceding Claims, wherein each plaster is individually sealed in an impervious wrapper, to retain the aroma until use.

5. A device according to any of the preceding Claims, wherein the aroma is vanilla.

6. A device according to any of Claims 1 to 4 wherein the aroma is lavender.

7. A device according to any of Claims 1 to 4 wherein the aroma is that of pumpkin.

8. A device according to Claim 5 wherein the substance is a 50/50 mixture of Floral Vanillin (Ref. C 6031/E Flora by A. Algto Ltd) and denatured ethanol, applied at the rate of 20 kilos per 250m roll of lint (one metre width).

9. A device according to any of the preceding Claims wherein the aroma comprises a single, pure aroma.

10. A device according to any of Claims 1 to 8 wherein the aroma comprises a mixture of different aromas, diluted if necessary in either ethanol or any other suitable dilutant.

11. A method of manufacturing an adhesive plaster incorporating an aroma, for application to the skin, the method including the steps of:-
(a) impregnating lint or other similar material with a substance giving the desired aroma and allowing the impregnated lint to dry,
(b) applying the lint to an adhesive fabric backing,
(c) forming a series of holes in the adhesive fabric backing, and
(c) cutting the lint and fabric combination into individual plasters with a central hole in the fabric backing of each plaster..

12. A method according to Claim 11 wherein the lint is preferably impregnated whilst in the roll, and is then slit into rolls of the desired width.

13. A method according to Claim 11 or Claim 12 wherein there is included the step of applying an impervious layer to the exposed surface of the impregnated lint.

14. A method according to Claim 13 wherein the free side of this impervious layer is then coated with a suitable adhesive, and a pair of peelable "shields" applied thereto, which are intended to be peeled off before applying the plaster to the skin.

15. A method according to any of Claims 11 to 14 wherein there is included a final step of sealing the plasters individually into an impervious wrapper.

## Patentansprüche

1. Vorrichtung zum Freisetzen eines Aromas umfassend Mull oder ein ähnliches Material, welches mit der das gewünschte Aroma freisetzenden Substanz getränkt ist, wobei der Mull in ein selbstklebendes Pflaster oder Pflästerchen aufgenommen ist, zum Aufbringen auf die Haut, wobei diejenige Oberfläche des Pflasters, welche freiliegt, wenn das Pflaster getragen wird, ein mittiges Loch aufweist, durch welches das Aroma aus dem Mull austreten kann.

2. Vorrichtung nach Anspruch 1, wobei die Oberfläche des Mulls, die sonst in Kontakt mit der Haut kommen könnte, mit einer undurchlässigen Schicht bedeckt ist, um zu verhindern, daß der Aromastoff durch die Haut in den Körper eindringt.

3. Vorrichtung nach einem der vorhergehenden Ansprüche, wobei das Pflaster aus durchlässigem Gewebe hergestellt ist welches es ermöglicht, daß das Pflaster "atmet" und daß das Aroma durch das Gewebe hindurch abgegeben werden kann.

4. Vorrichtung nach einem der vorhergehenden Ansprüche, wobei jedes Pflaster einzeln in einer undurchlässigen Verpackung verpackt ist, um das Aroma bis zur Anwendung zu bewahren.

5. Vorrichtung nach einem der vorhergehenden Ansprüche, wobei das Aroma Vanille ist.

6. Vorrichtung nach einem der Ansprüche 1 bis 4, wobei das Aroma Lavendel ist.

7. Vorrichtung nach einem der Ansprüche 1 bis 4, wobei das Aroma Kürbis ist.

8. Vorrichtung nach Anspruch 5, wobei der Stoff eine 50/50 - Mischung aus floralem Vanillin (Ref. C 6031/E Flora von A. Algto Ltd) und vergälltem Ethanol ist, welcher in einer Menge von 20 Kilo pro 250m-Rolle auf Mull (Breite 1 Meter) aufgebracht ist.

9. Vorrichtung nach einem der vorhergehenden Ansprüche, wobei das Aroma ein einziges, reines Aroma umfaßt.

10. Vorrichtung nach einem der Ansprüche 1 bis 8, wobei das Aroma eine Mischung verschiedener Aromen, wenn nötig mit Ethanol oder irgendeinem anderen geeigneten Lösemittel verdünnt, umfaßt.

11. Verfahren zur Herstellung eines selbstklebenden Pflasters enthaltend ein Aroma, zum Aufbringen auf die Haut, welches die folgenden Schritte enthält:
(a) Tränken von Mull oder einem anderen ähnlichen Material mit einer das gewünschte Aroma freisetzenden Substanz und Trocknenlassen des getränkten Mulls,
(b) Aufbringen des Mulls auf ein selbstklebendes Trägergewebe,
(c) Ausbilden einer Reihe von Löchern in dem selbstklebenden Trägergewebe,
(c) Zerschneiden der Zusammensetzung aus Mull und Gewebe in einzelne Pflaster mit einem mittigen Loch in dem Trägergewebe jedes Pflasters.

12. Verfahren nach Anspruch 11, worin der Mull vorzugsweise im Ballen gewickelt getränkt und dann in Ballen mit der gewünschten Breite zerschnitten wird.

13. Verfahren nach Anspruch 11 oder 12, worin ein weiterer Verfahrensschritt vorgesehen ist, nämlich Anbringen einer undurchlässigen Schicht auf die freiliegende Oberfläche des getränkten Mulls.

14. Verfahren nach Anspruch 13, worin die freie Seite der undurchlässigen Schicht anschließend mit einem geeigneten Klebstoff beschichtet wird und darauf ein Paar abziehbarer "Schilde" aufgebracht werden, welche vor dem Aufbringen des Pflasters auf die Haut abzogen werden sollen.

15. Verfahren nach einem der Ansprüche 1 bis 14, worin als letzter Verfahrensschritt das Verpacken der einzelnen Pflaster in eine undurchlässige Verpackung vorgesehen ist.

## Revendications

1. Dispositif destiné à être utilisé pour dégager un arôme, le dispositif comprenant de la peluche ou un matériau similaire imprégné(e) de la substance produisant l'arôme souhaité, la peluche étant incorporée dans un pansement ou un timbre adhésif, destiné à être appliqué sur la peau, dans lequel la surface du pansement qui est exposée lorsque le pansement est porté comprend un trou central ménagé dans celui-ci, afin que l'arôme provenant de la peluche puisse s'échapper à travers.

2. Dispositif selon la revendication 1, dans lequel la surface de la peluche qui, autrement, serait en contact avec la peau, est recouverte d'une couche imperméable, afin d'empêcher la substance aromatique de pénétrer dans le corps par la peau.

3. Dispositif selon l'une quelconque des revendications précédentes, dans lequel le pansement est constitué en tissu perméable, ce qui permet au pansement de "respirer", et à l'arôme d'être dégagé à travers le tissu.

4. Dispositif selon l'une quelconque des revendications précédentes, dans lequel chaque pansement est scellé individuellement dans un emballage imperméable, afin de conserver l'arôme jusqu'à utilisation.

5. Dispositif selon l'une quelconque des revendications précédentes, dans lequel l'arôme est la vanille.

6. Dispositif selon l'une quelconque des revendications 1 à 4, dans lequel l'arôme est la lavande.

7. Dispositif selon l'une quelconque des revendications 1 à 4, dans lequel l'arôme est celui de la citrouille.

8. Dispositif selon la revendication 5, dans lequel la substance est un mélange 50/50 de Vanille Florale (réf. C 6031/E Flore de A. Algto Ltd) et d'éthanol dénaturé, appliqué selon une proportion de 20 kilos par rouleau de 250 m de peluche (d'un mètre de large).

9. Dispositif selon l'une quelconque des revendications précédentes, dans lequel l'arôme comprend un arôme pur, unique.

10. Dispositif selon l'une quelconque des revendications 1 à 8, dans lequel l'arôme comprend un mélange de différents arômes, dilués si nécessaire soit dans de l'éthanol, soit dans tout autre diluant adapté.

11. Procédé de fabrication d'un pansement adhésif incorporant un arôme, destiné à être appliqué sur la peau, le procédé comprenant les étapes qui consistent à :
(a) imprégner de la peluche ou tout autre matériau similaire avec une substance produisant l'arôme désiré et laisser la peluche imprégnée sécher,
(b) appliquer la peluche sur un support en tissu adhésif,
(c) former une série de trous dans le support en tissu adhésif, et
(d) découper le produit composé que forment la peluche et le tissu pour en faire des pansements individuels, avec un trou central dans le support en tissu de chaque pansement.

12. Procédé selon la revendication 11, dans lequel la peluche est, de préférence, imprégnée pendant qu'elle se trouve en rouleau, et est ensuite fendue en rouleaux de la largeur désirée.

13. Procédé selon la revendication 11 ou la revendication 12, dans lequel est comprise l'étape qui consiste à appliquer une couche imperméable sur la surface exposée de la peluche imprégnée.

14. Procédé selon la revendication 13, dans lequel la face libre de cette couche imperméable est ensuite enduite d'un adhésif adapté et on y applique une paire de "protections" détachables qui sont destinées à être détachées avant d'appliquer le pansement sur la peau.

15. Procédé selon l'une quelconque des revendications 11 à 14, dans lequel est comprise une étape finale de scellement des pansements individuels dans un emballage imperméable.
